# EUROPEAN PATENT APPLICATION

(11) **EP 1 679 078 A1**
(43) Date of publication of application: **12.07.2006**
(21) Application number: 05014490.6
(22) Date of filing: 04.07.2005
(51) Int. Cl.: A61K 35/64, A61P 17/02

(54) **A composition for treating chronic wounds**

(30) Priority: 06.01.2005 TR 200500061
(71) Applicant: BEDIR, Yahya, Jeddah 21425 (SA); HAVITCIOGLU, Hayri, 35315 Izmir (TR)
(72) Inventor: BEDIR, Yahya, Jeddah 21425 (SA); HAVITCIOGLU, Hayri, 35315 Izmir (TR)
(74) Representative: Dereligil, Ersin

(57) **Abstract**

The present invention relates to a chemical composition used in wound healing. The present invention more particularly relates to the use of natural shellac resin in insistent, namely non-healing wounds. Thanks to the present invention, a novel application field is provided by making use of the influences of natural resin application on angiogenesis and wound healing in diabetes and gangrened wounds, psoriasis, and similar dermal diseases.

## Description

### Technical Field

The present invention relates to a chemical composition employed in wound healing. Thanks to the present invention, a novel application field is provided by making use of the influences of natural resin application on angiogenesis and wound healing in diabetes- and gangrened-based wounds, psoriasis, and similar dermal diseases.

### Background of Invention

There are many articles there in the literature on wound healing, chronic wounds, and particularly on diabetic wounds. There are also many experimental studies there, besides the clinical researches directed to this field. Clinical researches rather report clinical outcomes of applying different materials. Incandela and Oe Sanctis, for instance, researched in their clinical study the acute and four-week influences of local treatment with Essaven gel Ue on wound healing in diabetic patients, and obtained positive outcomes.

Newton, in his clinical studies, applied a dermal replacement treatment with Dermagraft on patients with diabetic foot wounds, and detected an increase of about 72% in the microvascular blood flow. He attributed the cause of this increase to granulation tissue formation as a result of this treatment on angiogenesis and to vasodilatation in present vein structures.

Diabetic subject models have been frequently developed in creating experimental chronic wounds and affects of many systemic and topical applications have been researched. Positive results have been reported for topical Becaplermin applications in diabetic patients' local wound healings. As another example, Brian reported in his study with genetically diabetic mice that the systemic and local application of Leptin, a protein secreted by adiposities, demonstrated a positive influence on wound healing, but that this case might not be the stimulation of angiogenesis.

Wound healing-related mediators and cellular molecule studies also occupy a substantial place in such researches. In this context, great consideration is given to fibroblast growth factors and vascular endothelial growth factor. The fibroblast growth factor is reported to show an increase in wound healing. And the vascular endothelial growth factor is known to show an increase in angiogenesis. Okumura researched the affects of basic fibroblast growth factor (bFGF) on wound healing, and demonstrated the role of bFGF treatment in augmenting angiogenesis and granulation tissue in his study with genetically diabetic mice.

Yamamoto researched in his study with genetically diabetic mice the affects of SM-10902, a stable prostacyclin analog, on wound healing, and showed SM-10902 to enhance angiogenesis and thus neovascularization in peripheral circulation failure so as to positively affect the wound healing. Montesinos reported positive results on angiogenesis and wound healing with the adenosine Ue stimulation of adenosine A(2A) receptors in chronic wounds.

The necessary criteria in a normal wound healing process comprise adequate oxygenation, properly-operating cellular functions, and a clean wound without bacterial contamination, and other many such known or unknown criteria. Under these conditions, primary wound healing will occur with minimal scaring *(sanatio per primam intenionem).* In coagulation, inflammatory, and granfilation phases, and scar formation periods of wound healing; blood cells with dynamic, interactive solved mediators, and extracellular matrix and parenchymal cells are to work. Cell synthesis and degradation are in equilibrium during the tissue remodelation phase of scar formation period, and normal healing. Concerning the chronic wounds (diabetic wound, venous ulcer, pressure wounds, etc), an extended inflammatory phase is mentionable. In such wounds, an increase is observed in cytokines working in tissue necrosis, infection, tissue hypoxia, and inflammation.

Some factors negatively influencing the process and event of wound healing are advanced age, nutritional disorders, presence of metabolic diseases (diabetes, uremia, etc), medicament use (corticosteroids, cytotoxic agents, etc), and radiation. In addition, the most significant local factor affecting wound healing is doubtlessly tissue perfusion. As a result of the decline in phagocytic activity together with the reduction in tissue perfusion, the tendency towards bacterial invasion and wound site infection enhances. A huge number of experimental and clinical researches are present in the literature for treating chronic wounds observed in conjunction with tissue perfusion defects. Many of such researches examine the extracellular matrix and cellular mechanisms or healing tissue with mechanical resistance methods on molecular, biochemical, histopathologic or genetic basis, whereby various methods or substances to ensure or enhance angiogenesis are studied.

The applications for achieving an efficient and swift wound healing process could not be completely succeeded in the above given researches so far. Therefore, the necessity for an application to be utilized in wound healings for accelerating the healing process has not yet been met completely.

### Brief Description of Invention

Regarding the present background of the relevant scientific technique, the main objective of the present invention is to provide a local wound-healing anti-gangrene composition to be used in problematic and delayed wound healings.

Another objective of the present invention is to avoid the excision of extremities such as hands or feet of thousands of patients due to such wounds, thereby maintaining their functions, and reducing the manpower and economical losses, and related psychological problems, due to such extremity losses.

A further objective is to ensure a composition applicable especially to dermal diseases such as psoriasis.

In order to meet said objectives and solve the problems experienced in the relevant art, the present invention provides a novel application field by making use of the affects of natural resin application on angiogenesis and wound healing in diabetes-based chronic wounds.

In this context and thanks to the present invention, natural shellac resin is made use of in insistent (i.e. non-healing) wounds, and the wound healing process is significantly achieved.

The shellac dogs polymer is obtained from Lac spider in a tree variety grown in China, India, and Thailand. Approximately 300.000 spiders are required to obtain one kilogram Lac. A novel and efficient application field is provided with the present invention for this material, which is known to ensure an easiness for humans to swallow pharmacologic tablets, once coated with this material in the form of a film coating.

The shellac raw material is quite simply handled: spiders are agglomerated and subjected to a thermal process. Whilst it is known to be a safe application, however, the exact composition of this material remains still unknown.

Shellac is a natural, complex polymeric material composed of polyester mixtures. Despite some small components of shellac are determined by chemical and derivation processes, the polymers have not yet been identified. Recent researches, however, suggest that the shellac content varies from monomer to tetramer acids, and that the main identifiable polyester is trihydro fatty acid and dihydroxyterponoid, known as aleuritic acid and jalarik acid, occupying one third of the whole content.

In brief, shellac is a mixture mainly composed of high molecular weight esters and acidic components. It can be shaped by compression under 121-134°C temperature and 6.9-24 MPa pressure. It can be shaped by cooling-down to 32-40°C without relieving the pressure. It is enough plastic due to its thermocytic structure, whereas its mechanical and electrical resistance can be enhanced with thermal processes.

### Detailed Description of Invention

Natural shellac resin is brought into a solution with ethyl alcohol in various proportions under the present invention. The chemical content of a so-formed resin is shown below in Table 1, as an example.

**Table 1**

| Content | | Perc. (%) |
|---|---|---|
| ■ | ester | 70-82 |
| ■ | free acid | 10-24 |
| ■ | free alcohol (max.) | 1 |
| ■ | hydrocarbons | 1-6 |

As described above, shellac is not yet exactly discovered concerning its content under the present technology; it is however a natural, complex material composed of polyester mixtures. Therefore, the aforementioned values are given only exemplary and by general findings for the composition under the present invention. A representational formula can be specified as C(60)H(90)O(15).

Said resin can be solved in basic, aliphatic, and aromatic solutions with alcohol, glycol ether, and acetic acid. The resin in alcoholic solutions releases the iron and directs the free iron.

The chemical and physical characteristics related to the resin is given in Table 2.

**Table 2**

| Characteristics | | Value |
|---|---|---|
| ■ | melting temperature (°C) | 70-82 |
| ■ | density (g/ml) | 0. 971 - 0. 980 |
| ■ | iodine value | 6.0 - 8.8 |
| ■ | acid value | 12.1 - 24.3 |
| ■ | saponification value | 63.8 - 83.0 |

Said resin, a thermoplastic material, can be given wide shape compositions with phenolic resin, and shaped at 12.1-13.4°C and 6.9-24 MPa. The shaping can be absorbed at 32-40°C without relieving the pressure.

Again, this resin can be carried within human body with resorbable materials like polylactic acid and polyglycolic acid and the like.

Thanks to the processes applied after the resin is melted with ethyl alcohol, it is completely cleaned with water, and separated from oil and other solid materials after centrifuging. Once such solid materials are precipitated, shellac natural resin floatable within the subject solution is obtained. This composition is refined from wax and solid materials.

### An Exemplary Application of the Present Invention

The subject natural shellac resin is brought into a solution with ethyl alcohol in a proportion of 100:125 by weight, and once the reaction is completed, 225 units of water is added so as all solid paraffin (wax) particles are separated.

Before such obtained solution is applied to a desired wound site, the site is well cleaned and bandaged in the form of several layers preferably with the use of a cotton dressing. Then the site of application is enclosed so as not to permeate air and to avoid leakage. As the simplest example, these conditions can be fulfilled by using a plastic bag as the coating material. After the solution poured into the bag is made interact with the wound site preferably for 8 hours, the coating material is removed. Said application manner, together with other medical treatments, is realized by airing the site for 15 minutes once in 8-hour periods, and then the relevant medicament is applied to such site. If the result of the culture test is negative, there remains no need for further medicating.

While all inanimate tissues became black as a result of the tests performed, animate tissues demonstrated a red color. Again in pathologic tests realized, it has been proved that the subject solution can be safely applied to gangrene and diabetes-based chronic wounds without imposing any side affect.

Especially the studies on experimental mice yielded 100% success. The subject solution acts as an initiating agent in the wound healing process of tissues near the gangrened site. It has surprisingly been found that such wounds healed in the first weak of the subject application.

## Claims

1. A chemical composition used in healing chronic wounds and dermal diseases, said composition being **characterized in** comprising natural shellac resin.

2. A composition according to Claim 1, said composition being **characterized in** further comprising ethyl alcohol.

3. A composition according to Claim 1, said wounds being preferably diabetes-based chronic wounds, gangrened wounds, and said dermal diseases being psoriasis and the like.

4. Use of a solution obtained from natural shellac resin and ethyl alcohol in treating diabetes-based chronic wounds, gangrened wounds, psoriasis, and similar dermal diseases.

5. A use method according to Claim 4, wherein paraffin particles formed in said solution are removed by the use of water.

6. A use method according to Claim 4, wherein said solution is applied to the site of chronic wound with a coating material providing air and water impermeability.
